# EUROPÄISCHE PATENTANMELDUNG

(11) **EP 1 172 078 A2**
(43) Veröffentlichungstag der Anmeldung: **16.01.2002**
(21) Anmeldenummer: 01116500.8
(22) Anmeldetag: 07.07.2001
(51) Int. Cl.: A61K 7/032

(54) **Mascara und Augenbrauenstifte mit einem Gehalt an Elastofasern**

(30) Priorität: 11.07.2000 DE 10033527
(71) Anmelder: Beiersdorf AG, 20245 Hamburg (DE)
(72) Erfinder: Lanzendörfer, Ghita, Dr., 22087 Hamburg (DE); Baum, Katja, Dr., 20257 Hamburg (DE); Bormann, Angelika, 22041 Hamburg (DE); Reuter, Bettina, 25451 Quickborn (DE); Hahn, Holger, Dr., 22397 Hamburg (DE)

(57) **Zusammenfassung**

Mascara- und/oder Augenbrauenstiftzubereitungen, dadurch gekennzeichnet, daß sie Elastofasem enthalten.

## Beschreibung

Die vorliegende Erfindung betrifft kosmetische Mascara und Augenbrauenstifte mit einem Gehalt an Substanzen, die die Wimpern oder die Augenbrauen, pflegen, schützen und zu deren Verschönerung dienen.

Der ganze menschliche Körper mit Ausnahme der Lippen, der Handinnenflächen und der Fußsohlen ist behaart, zum Großteil allerdings mit kaum sichtbaren Wolihärchen. Wegen der vielen Nervenenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares und der Wimpern und Augenbrauen dürfte allerdings heute darin bestehen, das Aussehen des Menschen in charakteristischer Weise mitzugestalten. Ähnlich wie die Haut erfüllen sie eine soziale Funktion, da sie über ihr Erscheinungsbild erheblich zu zwischenmenschlichen Beziehungen und zum Selbstwertgefühl des Individuums beitragen.

Die Haare, d.h. auch die Augenbrauen und Wimpern, bestehen aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar ständig neu gebildet wird. Der Haarschaft seinerseits ist aus drei Schichten aufgebaut: einem zentralen Teil - dem sogenannten Haarmark (Medulla), welches allerdings beim Menschen zurückgebildet ist und oft gänzlich fehlt - ferner dem Mark (Cortex) und der äußeren, bis zu zehn Lagen starken Schuppenschicht (Cuticula), die ein ganzes Haar umhüllt.

Das Färben des menschlichen Haupthaares und insbesondere der Augenbrauen und Wimpern ist ein wichtiges Gebiet der dekorativen Kosmetik. Eine dauerhafte Färbung, wie es auch beim Haupthaar oftmals erwünscht ist, sollte auch durch intensives Waschen nicht wieder aus dem Haar entfernbar sein und in Hinsicht auf Umwelteinflüsse wie UV- Licht, Schweiß und Feuchtigkeit eine hohe Echtheit aufweisen. Entsprechende Färbemittel werden in der Regel auf Basis von Oxidationsfarbstoffvorprodukten formuliert.

Im Rahmen moderner Modetrends besteht aber immer häufiger das Bedürfnis nach Farbeffekten am Haar, die auch ohne Inanspruchnahme eines Friseurs oder Kosmetikers auf einfache Weise erzielbar und ebenso leicht, z. B. durch einmaliges, übliches Waschen des Haares, wieder zu entfernen sind. Insbesondere sollen sich die entsprechenden Farbeffekte auch auf einzelne Haarsträhnen oder Haarpartien beschränken lassen. Weiterhin ist es in hohem Maße wünschenswert, wenn sich die auch noch auf der fertig gestylten Frisur anbringen lassen.

Eine Produktgruppe, die prinzipiell diese Anforderungen erfüllen könnte, stellen die Wimpemtuschen dar, die auch unter dem Namen "Mascara" (wohl letztlich von arab.: 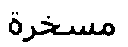 (mashara) = Possenreißer, Harlekin, auch: Harlekinade; vgl.: Maskerade), bekannt sind. Augen Make-up, insbesondere Mascaraprodukte (Wimperntusche), haben im dekorativen Marktsegment eine große Bedeutung. Mit Mascara, aber auch mit Augenbrauenstiften, kann man die Wimpern und Brauen optisch effektvoll betonen und den Augen dadurch mehr Ausdruck verleihen.

Die Qualität einer Mascara lässt sich messen an einem guten und leichten Auftrag, einer angemessenen Trockenzeit und einer guten Haftfestigkeit. Weiterhin sollte eine Mascara sich nicht nachteilig auf das Wimpernhaar auswirken, z.B. nicht austrocknend wirken oder die Brüchigkeit erhöhen. Im Gegenteil: Es ist wünschenswert, eine Mascara zu erhalten, die neben den genannten Aspekten sogar noch pflegende Eigenschaften aufweist und den Wimpern Schwung und Elastizität verleiht.

Ein großes Problem stellen dabei jedoch die Haftfestigkeit der Mascara auf dem Haar/den Wimpern dar. Die Konsistenz, die Trockenzeit und der auf der Wimper verbleibende Film einer Mascara muss so beschaffen sein, daß für ein gute optische Betonung ausreichend Masse auf jeder Wimper haften bleibt, mehrere Wimpern jedoch nicht zu sogenannten "Fliegenbeinen" zusammenkleben. Insbesondere sollte auch nach mehreren Stunden Tragezeit der getrocknete Film nicht "bröckelig" werden und sich nicht durch Feuchtigkeit, Sebum oder z.B. anderen öllöslichen Bestandteile einer Pflegecreme anlösen lassen, so daß sich keine dunklen Ränder um die Augen bilden. Durch die Einstellung der Trockenzeit wird die Zeit definiert, die der Anwenderin zum (auch mehrmaligen) Auftragen bleibt, bevor der Mascarafilm getrocknet / fixiert ist.

Dabei ist der Einsatz eines Filmbildners zur Steuerung der vorgenannten Eigenschaften von besonderer Bedeutung.

Die Verwendung von Filmbildnern in haarkosmetischen Zubereitungen, insbesondere Mascarazubereitungen, ist an sich bekannt. Die DE-OS 197 46 468 beschreibt eine Mascara, enthaltend mindestens ein Weiß- oder Farb-Pigment sowie übliche kosmetische Bestandteile in einer wässrigen Grundlage, dadurch gekennzeichnet, daß sie ein Wachs mit einem Schmelzpunkt von mindestens 60 °C und ein nichtionogenes synthetisches Polymer enthält. Die am angegebenen Ort als bevorzugt dargestellten Polymere werden beispielsweise gewählt aus der Gruppe der nichtionogenen, synthetischen Polymere, insbesondere Polyvinylalkohole, Poly(N-vinyl-formamid), Poly(N-vinylcaprolactam), Polyvinylpyrrolidone sowie Copolymere des Vinylpyrrolidons mit mindestens einem weiteren nichtionogen Monomer. Als bevorzugt werden Vinylacetat Polyvinylalkohole sowie Polyvinylpyrrolidone, Copolymere und Mischungen dieser Polymeren dargestellt.

Weiterhin ist bei der Verwendung von Mascara erwünscht, daß den Wimpern ein Schwung verliehen wird und nach der Antrocknung des Maskaras die Wimpern elastisch bleiben.

Aufgabe der vorliegenden Erfindung war, die Nachteile des Standes der Technik zu beseitigen. Insbesondere sollten verbesserte kosmetische Zubereitungen zur Verfügung gestellt werden, die sich durch besseren Filmbildung auszeichnen, indem der getrocknete Film elastische und doch formgebende Eigenschaften aufweist. Insbesondere ist jedoch die Verbesserung der Haftfestigkeit in Bezug auf Wasser- und Wischfestigkeit Aufgabe der vorliegenden Erfindung.

Weiterhin sollten Zubereitungen zur Verfügung gestellt werden, die sich nicht nachteilig auf das Wimpemhaar auswirken, z.B. nicht austrocknend wirken oder die Brüchigkeit erhöhen.

Erfindungsgemäß werden diese Aufgaben gelöst und die Nachteile des Standes der Technik beseitigt.

Gegenstand der Erfindung sind Mascara und Augenbrauenstifte, dadurch gekennzeichnet, daß sie Elastofasern enthalten.

Elastofasern sind Chemiefasern, die äußerst dehnbar sind und nach Aufhebung der Zugkraft weitgehend in den ursprünglichen Zustand zurückkehren. Die wichtigsten Vertreter sind Elastan (früher Elasthan, Kurzzeichen: EL; in den USA ist die Bezeichnung Spandex üblich), Fasern aus Hochpolymeren, die zu mindestens 85 Gew.-% aus segmentiertem Polyurethan bestehen, und Elastodien (Kurzzeichen: ED), Fasern, die aus synthetischem Polyisopren oder aus Hochpolymeren bestehen, die durch Polymerisation eines oder mehrerer Diene, eventuell unter Zusatz eines oder mehrerer Vinylmonomerer, entstanden sind. Zur zweiten Gruppe kann man auch die Gummifasern (Kurzzeichen: LA) aus Naturkautschuk zählen. Elastodiene werden häufig vulkanisiert. Elastische Eigenschaften besitzt auch eine Bikomponentenfaser aus Polyamid und Polyurethan.

Ein großer Teil der heute produzierten Elastomerfasern wird aus vulkanisierten Kautschuken gefertigt. Daneben haben die sogenannten Spandex-Fasern große Bedeutung erlangt. Diese bestehen aus Blockcopolymeren, die durch die Reaktion von Diisocyanaten mit linearen, Hydroxy-Gruppen-terminierten Oligomeren und Kettenverlängerern (Diamine oder Glykole) hergestellt werden:

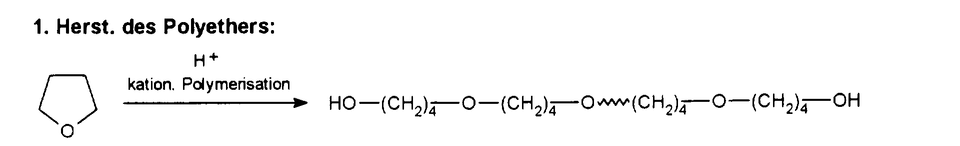

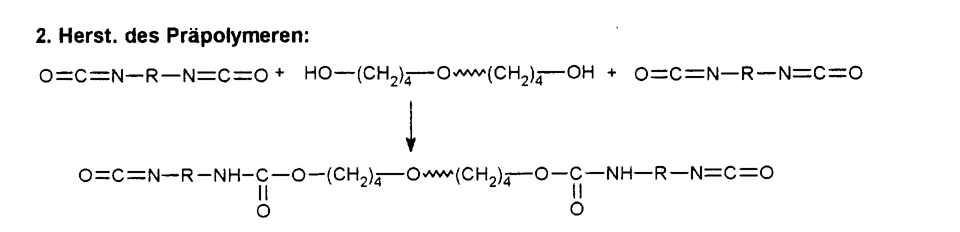

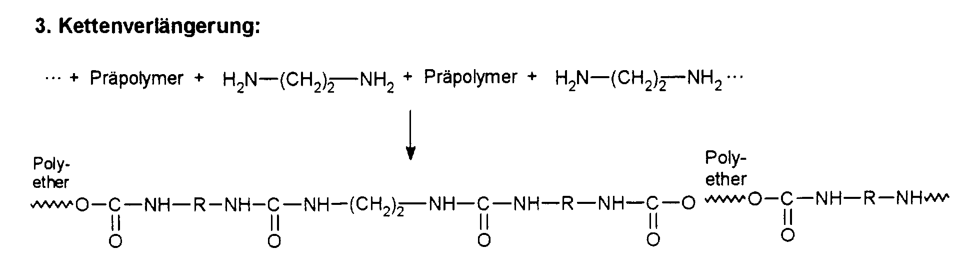

Die so erhaltenen thermoplastischen Elastomere werden anschließend durch Schmelz- od. Lösungsspinnen verarbeitet. In den resultierenden Spandex-Fasern wirken die "harten" Polyurethan-Segmente als physikalische Vemetzungsbereiche für die weiche Matrix aus Polyether-Segmenten. Spandex-Fasern weisen etwa die gleiche Reißdehnung und die gleiche Reißfestigkeit wie Gummifäden auf, haben aber etwa doppelt so hohe Elastizitätsmoduln und ein höheres Feuchteaufnahme- und Wasserrückhalte-Vermögen. Sie werden allerdings in der Regel nicht in reiner Form, sondern in Kombination mit anderen Fasern eingesetzt.

Erfindungsgemäß bevorzugt sind solche Elastofasern, die von der Gesellschaft DuPont unter der Bezeichnung Lycra® verkauft werden.

Die Elastofasern sind in den erfindungsgemäßen Zubereitungen bevorzugt in Mengen von 0,01 - 50,0 Gew.-%, bezogen auf das Gesamtgewicht der Zubereitungen, enthalten. Mengen von 0,1 - 20,0 Gew.-%, insbesondere von 0,2 - 10,0 Gew.-%, sind besonders bevorzugt.

Lycra ® ist eine hochelastische textile Faser, die aus mindestens 85 Gew. % aus segmentierten Polyurethan besteht und die unter Einwirkung einer Zugkraft um das dreifache der ursprüngliche Länge gedehnt, nach Entlastung sofort wieder nahezu in die Ausgangslage zurückkehren. Ähnliche Eigenschaften finden sich bei den Fasern Dorlastan®, Elastan oder Spandex ®.

Die Elastofasern können in die Öl- oder Wasserphase der Maskaraformulierung eingesetzt werden. Vorteilhaft werden die Elastofasern, bevorzugt Lycra ®, in einer Länge von 0,01 bis 1 cm eingesetzt, besonders vorteilhaft ist eine Länge von 0,05 bis 0,5 cm.

Die Zubereitungen, die erfindungsgemäße Wirkstoffkombinationen enthalten, sind topische Zubereitungen. Diese können wie üblich zusammengesetzt sein und zur Behandlung und der Pflege der Wimpern und Augenbrauen oder der die umgebenden Haut dienen. Zur Anwendung werden die erfindungsgemäßen Zubereitungen in der üblichen Weise in ausreichender Menge aufgebracht.

Vorteilhaft können Zubereitungen im Sinne der vorliegenden Erfindung beispielsweise als Blockmascara, Mascara in Cremeform (wasserfrei oder emulgiert), oder als Augenbrauenstifte, z.B. als "Crayons" oder "Pencil" vorliegen.

Die erfindungsgemäßen Wirkstoffkombinationen können in allen üblichen Wimperntuschen, Mascara und Augenbrauenstift-Zubereitungen verwendet werden.

Vorteilhaft enthalten erfindungsgemäße Zubereitungen neben einem wirksamen Gehalt an erfindungsgemäßen Wirkstoffkombinationen ferner übliche Farb-, Wirk-, Inhalts-, Zusatz- und/oder Hilfsstoffe.

Präparate vom "Cake"-Typ (Block-Mascara) bestehen aus einer Mischung von Farbstoff, Fetten und Wachsen und Öl-in-Wasser-Emulgatoren. Als Emulgator verwendet man zumeist Triethanolaminstearat.

Beim Auftragen des Präparates verwendet man eine feuchte Bürste. Durch das anwesende Wasser bildet sich an der Oberfläche des Mascarablockes eine Öl-in-Wasser-Emulsion. Diese wird von der Bürste aufgenommen. Auf den Wimpern trocknet sie wieder ein.

Wasserfreie Mascara sind überwiegend gelförmige Zubereitungen auf Öl-Wachsbasis mit einem hohen Anteil flüchtiger Lösungsmittel.

Wasserhaltige, emulgierte Mascara in Cremeform besitzen als Basis z.B. eine Öl-in-Wasser-Creme vom Stearat- oder Glycerylmonostearattyp.

Bevorzugte Emulsions-Mascara im Sinne der vorliegenden Erfindung enthalten als Hauptbestandteile beispielsweise synthetische und/oder natürliche Wachse,synthetische und/oder natürliche Öle, wie z.B. Triglyceride der Linolsäure, O/W-Emulgatoren, vorzugsweise Stearinsäure, neutralisiert mit TEA oder NaOH, und ggf. einen Coemulgator, mehrwertige Alkohole, vorzugsweise Butandiol oder Propandiol, Pigmente und / oder Perlglanzpigmente (Eisenoxide, Ultramarinblau, Chromhydroxidgrün), Füllstoffe, vorzugsweise Talkum, Kaolin oder Mica und gegebenenfalls auch Hilfsstoffe.

Gegebenenfalls können zu den eingesetzten erfindungsgemäßen sulfonierten Elastofasern, insbesondere Lycra ® auch ein oder mehrere weitere wasserlösliche Filmbildner, z.B. aus der Klasse der PVP/VA-Copolymere oder Ammoniumacrylate zugegeben werden.

Nach Festlegung der Deutschen Gesellschaft für Fettwirtschaft (*Fette, Seifen*, *Anstrichmittel*, ***76***, *135 [1974]*) werden zur Kennzeichnung des Begriffes "Wachs" in der Regel die mechanisch-physikalischen Eigenschaften der Wachse, welche für ihre Verwendung maßgebend sind, herangezogen, während für die Begriffsbestimmung die jeweilige chemische Zusammensetzung unberücksichtigt bleibt.

"Wachs" ist - ähnlich wie "Harz" - eine Sammelbezeichnung für eine Reihe natürlicher oder künstlich gewonnener Stoffe, die in der Regel folgende Eigenschaften aufweisen: bei 20 °C knetbar, fest bis brüchig hart, grob- bis feinkristallin, durchscheinend bis opak, jedoch nicht glasartig, über 40 °C ohne Zersetzung schmelzend, schon wenig oberhalb des Schmelzpunkts verhältnismäßig niedrigviskos und nicht fadenziehend, stark temperaturabhängige Konsistenz und Löslichkeit und unter leichtem Druck polierbar. Ist in Grenzfällen bei einem Stoff mehr als eine der vorstehend genannten Eigenschaften nicht erfüllt, so ist er kein Wachs im Sinne dieser Definition. Wachse unterscheiden sich von ähnlichen synthetischen oder natürlichen Produkten (z. B. Harzen, plastischen Massen usw.) hauptsächlich darin, daß sie in der Regel etwa zwischen 50 und 90 °C, in Ausnahmefällen auch bis zu etwa 200 °C, in den schmelzflüssigen, niedrigviskosen Zustand übergehen und praktisch frei von aschebildenden Verbindungen sind.

Vorteilhaft im Sinne der vorliegenden Erfindung sind außerdem Esterwachse, die Ester aus
1. gesättigten und/oder ungesättigten, verzweigten und/oder unverzweigten Mono- und/oder Dicarbonsäure mit 10 bis 50 Kohlenstoffatomen, bevorzugt 15 - 45 Kohlenstoffatomen und
2. Glycerin
darstellen.

Die nachfolgend aufgelisteten Wachse können vorteilhaft im Sinne der vorliegenden Erfindung verwendet werden:

| **Klasse** | **Untergruppe** | **Beispiele** |
|---|---|---|
| natürliche Wachse | pflanzliche Wachse | Candelillawachs, Carnaubawachs, Japanwachs, Espartograswachs, Korkwachs, Guarumawachs, Reiskeimölwachs, Zuckerrohrwachs, Ouricurywachs, Montanwachs, Jojobawachs, Sonnenblumenwachs |
| | tierische Wachse | Bienenwachs, Schellackwachs, Walrat, Lanolin (Wollwachs), Bürzelfett |
| | Mineralwachse | Ceresin, Ozokerit (Erdwachs) |
| | petrochem. Wachse | Petrolatum, Paraffinwachse, Mikrowachse |
| chem. modifizierte Wachse | Hartwachse | Montanesterwachse, Sasolwachse, hydrierte Jojobawachse |
| synthet. Wachse | | Polyalkylenwachse, Polyethylenglykolwachse |

Es ist ferner vorteilhaft, die Wachskomponenten aus der Gruppe der Glyceride, insbesondere aus der Gruppe der Triglyceride zu wählen. Besonders vorteilhaft sind die im folgenden aufgelisteten Glyceride und Triglyceride:

| **Glycerid** | **Handelsname** | **erhältlich bei** |
|---|---|---|
| C₁₆₋₁₈-Triglycerid | Cremeol HF-52-SPC | Aarhus Oliefabrik |
| Glycerylhydroxystearat | Naturchem GMHS | Rahn |
| Hydrierte Coco-Glyceride | Softisan 100 | Hüls AG |
| Caprylisäure/Caprinsäure/lsostearinsäure/ | Softisan 649 | Dynamit Nobel |
| Adipinsäure Triglycerid C₁₈₋₃₆ Triglycerid | Syncrowax HGLC | Croda GmbH |
| Glyceryltribehenat | Syncrowax HRC | Croda GmbH |
| Glyceryl-tri-(12-hydroxystearat) | Thixcin R | Rheox/NRC |
| Hydriertes Ricinusöl | Cutina HR | Henkel KGaA |
| C₁₆₋₂₄-Triglycerid | Cremeol HF-62-SPC | Aarhus Oliefabrik |

Bevorzugtes Glycerid im Sinne der Erfindung sind C₁₈₋₃₆ -Triglyceride.

Es ist von Vorteil den Gehalt an Wachsen in erfindungsgemäßen Zubereitungen aus dem Bereich von 0,01-10 Gew.-%, bevorzugt 0,1-5 Gew.-% zu wählen.

Erfindungsgemäße, als Emulsionen vorliegende Zubereitungen enthalten einen oder mehrere Emulgatoren. 0/W-EMulgatoren können beispielsweise vorteilhaft gewählt werden aus der Gruppe der polyethoxylierten bzw. polypropoxylierten bzw. polyethoxylierten und polypropoxylierten Produkte, z.B.:
- der Fettalkoholethoxylate
- der ethoxylierten Wollwachsalkohole,
- der Polyethylenglycolether der allgemeinen Formel R-O-(-CH₂-CH₂-O-)ₙ-R',
- der Fettsäureethoxylate der allgemeinen Formel R-COO-(-CH₂-CH₂-O-)ₙ-H,
- der veretherten Fettsäureethoxylate der allgemeinen Formel R-COO-(-CH₂-CH₂-O-)ₙ-R',
- der veresterten Fettsäureethoxylate der allgemeinen Formel R-COO-(-CH₂-CH₂-O-)ₙ-C(O)-R',
- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel R-O-(-CH₂-CH₂-O-)ₙ-CH₂-COOH,
- der Polyoxyethylensorbitolfettsäureester,
- der Alkylethersulfate der allgemeinen Formel R-O-(-CH₂-CH₂-O-)ₙ-SO₃-H
- der Fettalkoholpropoxylate der allgemeinen Formel R-O-(-CH₂-CH(CH₃)-O-)ₙ-H,
- der Polypropylenglycolether der allgemeinen Formel R-O-(-CH₂-CH(CH₃)-O-)ₙ-R',
- der propoxylierten Wollwachsalkohole,
- der veretherten Fettsäurepropoxylate R-COO-(-CH₂-CH(CH₃)-O-)ₙ-R',
- der veresterten Fettsäurepropoxylate der allgemeinen Formel R-COO-(-CH₂-CH(CH₃)-O-)ₙ-C(O)-R',
- der Fettsäurepropoxylate der allgemeinen Formel R-COO-(-CH₂-CH(CH₃)-O-)ₙ-H,
- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel R-O-(-CH₂-CH(CH₃)O-)ₙ-CH₂-COOH
- der Alkylethersulfate bzw. die diesen Sulfaten zugrundeliegenden Säuren der allgemeinen Formel R-O-(-CH₂-CH(CH₃)-O-)ₙ-SO₃-H
- der Fettalkoholethoxylate/propoxylate der allgemeinen Formel R-O-Xₙ-Yₘ-H,
- der Polypropylenglycolether der allgemeinen Formel R-O-Xₙ-Yₘ-R',
- der veretherten Fettsäurepropoxylate der allgemeinen Formel R-COO-Xₙ-Yₘ-R',
- der Fettsäureethoxylate/propoxylate der allgemeinen Formel R-COO-Xₙ-Yₘ-H,.

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 18, ganz besonders vorteilhaft mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:
Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17), Polyethylenglycol(18)stearylether (Steareth-18), Polyethylenglycol(19)stearylether (Steareth-19), Polyethylenglycol(20)stearylether (Steareth-20),
Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth19), Polyethylenglycol(20)isostearylether (Isosteareth-20),
Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20),
Polyethylenglycol(13)isocetylether (lsoceteth-13), Polyethylenglycol(14)isocetylether (Isoceteth-14), Polyethylenglycol(15)isocetylether (lsoceteth-15), Polyethylenglycol(16)isocetylether (Isoceteth-16), Polyethylenglycol(17)isocetylether (Isoceteth-17), Polyethylenglycol(18)isocetylether (Isoceteth-18), Polyethylenglycol(19)isocetylether (Isoceteth19), Polyethylenglycol(20)isocetylether (lsoceteth-20),
Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15),
Polyethylenglycol(12)laurylether (Laureth-12), Polyethylenglycol(12)isolaurylether (lsolaureth-12).

Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol(19)cetylstearylether (Ceteareth-19), Polyethylenglycol(20)cetylstearylether (Ceteareth20),

Es ist femer von Vorteil, die Fettsäureethoxylate aus folgender Gruppe zu wählen:
Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat,
Polyethylenglycol(12)isostearat, Polyethylenglycol(13)isostearat, Polyethylenglycol(14)isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol(22)isostearat, Polyethylenglycol(23)isostearat, Polyethylenglycol(24)isostearat, Polyethylenglycol(25)isostearat,
Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat
Als ethoxylierte Alkylethercarbonsäure bzw. deren Salz kann vorteilhaft das Natriumlaureth-1 1-carboxylat verwendet werden.

Als Alkylethersulfat kann Natrium Laureth 1-4 sulfat vorteilhaft verwendet werden.

Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlänge von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12 - 18 C-Atomen, Diglycerinether gesättigter und/oder ungesättigter, verzweigter und/oder unverzweigter Alkohole einer Kettenlänge von 8 bis 24, insbesondere 12 - 18 C-Atomen, Propylenglycolester gesättigter und/oder ungesättigter, verzweigter und/oder unverzweigter Alkancarbonsäuren einer Kettenlänge von 8 bis 24, insbesondere 12 - 18 C-Atomen sowie Sorbitanester gesättigter und/oder ungesättigter, verzweigter und/oder unverzweigter Alkancarbonsäuren einer Kettenlänge von 8 bis 24, insbesondere 12 - 18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Es kann auch vorteilhaft sein, daß die erfindungsgemäßen Mascarazubereitungen ein oder mehrere Pigmente enthalten, gewählt aus der Gruppe der Pigmente mit den C.I.-Bezeichnungen Pigment Red 57 : 1, Pigment Red 57 : 2. Pigment Red 172, Pigment Red 90 : 1, Pigment Yellow 100, Pigment Yellow 115, Pigment Red 174, Pigment Red 4, Pigment Blue 29, Pigment Violet 15, Pigment Violet 16, Pigment Red 29, Pigment Green 17, Pigment Green 18, Natural Red 4, Pigment White 6, Pigment White 14 und Pigment White 31. Pigment Blue 29, Pigment Violet 15, Pigment Violet 16, Pigment Green 18, Natural Red 4, Pigment White 6 und Pigment White 31..

Die Mascarazubereitungen enthalten die Pigmente bevorzugt in Mengen von 2-15 Gew.%, insbesondere von 5,0-10,0 Gew.-%, jeweils bezogen auf die gesamte Zubereitung.

Gemäß einer bevorzugten Ausführungsform der Erfindung enthält die Haar-Mascara noch einen oder mehrere Weichmacher. Bevorzugte Weichmacher sind Polyethylenglykole, Polypropylenglykole, Polyglycerine sowie deren Mischungen. Besonders bevorzugt sind Polyethylenglykole mit mittleren Molmassen von 100 bis 4000, insbesondere 200 bis 1500. Polyethylenglykole mit mittleren Molmassen von 300 bis 600 sind erfindungsgemäß besonders bevorzugte Weichmacher. Aber auch Citronensäurealkylester und Acylcitronensäurealkylester wie Triethylcitrat, Tributylcitrat, Acetyltriethylcitrat und Acetyltributylcitrat können vorteilhaft Verwendung finden.

Es ist im Rahmen der Erfindung bevorzugt, daß die Weiß- und Farb-Pigmente die einzigen farbgebenden Komponenten der Mascara sind. Es soll jedoch prinzipiell nicht ausgeschlossen werden, daß zur Nuancierung geringe Mengen sehr gut auswaschbarer, direktziehender Farbstoffe in der Mascara enthalten sind.

Bevorzugte wasserfeste Mascara enthalten beispielsweise Gelbildner, z.B. Quatemium-18-hectorite (Bentone-38), Aktivator für den Gelbildner, z.B. Alkohole wie z.B. Ethanol, Verdicker, z.B. substituierte und unsubstituierte Cellulosen, Wachse zur Strukturgebung, öl- bzw. fettlösliche Filmbildner, z.B. PVP/Eicosene-Copolymer, Pigmente, wie z.B. vorstehend angegeben, Lösungsmittel, z.B. Kohlenwasserstoffe, vorzugsweise Kettenlänge C₉-C₁₂ und/oder flüchtige Silikone, z.B. Cyclomethicone und gegebenenfalls Hilfsstoffe.

Viele dekorative Augenprodukte, wie z.B. Augenbrauenpräparate, werden in Form von Stiften angeboten. Zum Nachzeichnen der Augenbrauen werden meist Formulierungen verwendet, bei denen die farbgebenden Pigmente in einer Wachs-/Öl-Basis eingelagert sind. Im allgemeinen wird die fettige Farbstoff-Wachsmasse als Mine extrudiert und in Stiften aus Rotzedernholz konfektioniert.

Augenbrauenstifte werden heute in zwei Formen verwendet: als gegossene Stifte und als "Pencils". Die Schminkstifte sind, dem Aufbau nach, harte Fettschminken. Geeignete Augenbrauenstifte des Standes der Technik mit einem Gehalt an Paraffinen und Bienenwachs bzw. Japanwachs sind in "Kosmetik, Entwicklung, Herstellung und Anwendung kosmetischer Mittel", Herausgeber: W. Umbach, Georg Thieme Verlag, Stuttgart-New York, 1995, S. 321 ff, beschrieben.

Die erfindungsgemäßen Zubereitungen können kosmetische Hauptkomponenten und Hilfsstoffe enthalten, wie sie üblicherweise in solchen Zubereitungen verwendet werden, z.B. Konservierungsmittel, Parfüme, Substanzen zum Verhindern des Schäumens, Schaumstabilisatoren, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, rückfettende Agentien, Fette, Öle, Wachse, Alkohole, Polyole und deren toxikologisch verträglichen Ether und Ester, verzweigte und/oder unverzweigte Kohlenwasserstoffe, weitere Antioxidantien, Stabilisatoren, pH-Wert-Regulatoren, Konsistenzgeber, Bakterizide, antimikrobielle Stoffe, UV-Absorber, Komplexierungs- und Sequestrierungsagentien, Perlglanzagentien, Polymere, Elektrolyte, organische Lösungsmittel, Silikonderivate, Pflanzenextrakte, Vitamine und/oder andere Wirkstoffe oder andere übliche Bestandteile einer kosmetischen oder dermatologischen Formulierung. Auch Lösungsvermittler, z.B. zur Einarbeitung hydrophober Komponenten wie z.B. von Parfümzubereitungen können enthalten sein.

Die Gesamtmenge der Hauptkomponenten beträgt beispielsweise 85 bis 99,999 Gew.%, vorzugsweise 90 bis 99,99 Gew.-%, jeweils bezogen auf das Gesamtgewicht der Zubereitung.

Die Gesamtmenge der Hilfsstoffe beträgt beispielsweise 0,001 bis 15 Gew.-%, vorzugsweise 0,01 bis 10 Gew.-%, jeweils bezogen auf das Gesamtgewicht der Zubereitung.

Die erfindungsgemäßen Zubereitungen können Wasser, z.B. 1 bis 80 Gew.-% und/oder Alkohole, z.B. 0,5 bis 10 Gew.-% enthalten, jeweils bezogen auf das Gesamtgewicht der Zubereitungen.

Erfindungsgemäß können als weitere Antioxidantien alle für kosmetische und/oder dermatologische Anwendungen geeigneten oder gebräuchlichen Antioxidantien verwendet werden.

Die Gesamtmenge der Antioxidantien beträgt beispielsweise 0,001 bis 10 Gew.-%, vorzugsweise 0,05 bis 5 Gew.-%, jeweils bezogen auf das Gesamtgewicht der Zubereitung.

Es ist auch von Vorteil, den erfindungsgemäßen Zubereitungen Antioxidantien zuzusetzen. Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), femer (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Herstellung der erfindungsgemäßen Zubereitungen kann in der üblichen Weise durch Mischen der einzelnen Bestandteile erfolgen. Die Wirkstoffe der erfindungsgemäßen Kombinationen oder auch die vorgemischten Bestandteile der erfindungsgemäßen Kombinationen können im Mischvorgang zugegeben werden.

Alle Mengenangaben, Anteile und Prozentanteile sind, soweit nicht anders angegeben, auf das Gewicht und die Gesamtmenge bzw. auf das Gesamtgewicht der Zubereitungen oder der jeweiligen Mischung bezogen.

Die folgenden Beispiele verdeutlichen die Erfindung.

### Beispiele 1 bis 5

| Emulsionsmascara | 1 | 2 | 3 | 4 | 5 |
|---|---|---|---|---|---|
| | Gew.-% | Gew.-% | Gew.-% | Gew.-% | Gew.-% |
| Stearinsäure | 3,50 | 4,00 | 4,00 | 4,00 | 3,50 |
| Ozokerit | 7,00 | 6,00 | 5,00 | 5,00 | 7,00 |
| Carnaubawachs | 3,00 | 3,50 | 3,50 | 3,50 | 3,00 |
| Neutralöl | 3,00 | 4,00 | - | 1,00 | 3,00 |
| Oleylalkohol | - | - | 3,00 | - | - |
| Sorbitansesquioleat | 1,50 | 1,80 | 1,80 | 1,50 | 1,50 |
| Triethanolamin | 1,00 | 1,10 | 1,10 | 1,10 | 1,00 |
| Lycra® | 0,05 | 0,50 | 0,75 | 1,00 | 0,50 |
| PVP Copolymer | - | - | - | - | 3,00 |
| Butandiol | 2,00 | 1,50 | 1,40 | 1,50 | 2,00 |
| Talkum | 5,00 | 3,00 | - | 2,00 | 5,00 |
| Farbpigmente (Eisenoxide) | 8,00 | 9,00 | 10,00 | 9,00 | 8,00 |
| Calciumpanthotenat | 1,30 | 4,80 | 0,10 | 0,50 | 1,30 |
| Gamma-Oryzanol | 2,30 | 0,20 | 5,00 | 2,00 | 2,30 |
| Konservierungsmittel | q.s. | q.s. | q.s. | q.s. | q.s. |
| Wasser, demineralisiert | ad 100,00 | ad 100,00 | ad 100,00 | ad 100,00 | ad 100,00 |

### Beispiele 6 bis 8

| Wasserfeste Mascara | 6 | 7 | 8 |
|---|---|---|---|
| | Gew.-% | Gew.-% | Gew.-% |
| Quaternium-18-Hectorite | 10,00 | 12,00 | 10,00 |
| Talkum | 5,00 | 4,00 | 5,00 |
| Ethanol | 2,50 | 2,75 | 2,50 |
| Lycra ® | 0,05 | 0,40 | 1,00 |
| Carnaubawachs | 2,00 | - | 3,00 |
| PVP/Eicosencopolymer | 20,00 | 18,00 | 17,00 |
| Perlglanzpigmente | 5,00 | 3,00 | 3,00 |
| Farbpigmente(Eisenoxide) | 10,00 | 10,00 | 10,00 |
| Nylon-6 | - | 2,00 | 1,00 |
| Cyclomethicon | 5,00 | - | 3,00 |
| Konservierungsmittel | q.s. | q.s. | q.s. |
| Calciumpanthotenat | 0,80 | 0,10 | 0,20 |
| Gamma-Oryzanol | 0,20 | 5,00 | 1,00 |
| Isoparaffin C₁₀₋₁₂ | ad 100,00 | ad 100,00 | ad 100,00 |

## Patentansprüche

1. Mascara- und/oder Augenbrauenstiftzubereitungen, **dadurch gekennzeichnet, daß** sie Elastofasern enthalten.

2. Zubereitungen nach Anspruch 1, **dadurch gekennzeichnet, daß** die polymere Hauptkette der Elastofasem gewählt wird aus der Gruppe der segmentierten Polyurethane.

3. Zubereitungen nach Anspruch 1 oder Verwendung nach Anspruch 3, **dadurch gekennzeichnet, daß** der Gehalt an Elastofasem in Mengen von 0,01 - 50,0 Gew.-%, bezogen auf das Gesamtgewicht der Zubereitungen , bevorzugt 0,1 - 20,0 Gew.-%, insbesondere von 0,2 - 10,0 Gew.-%, beträgt.
